(19) Europäisches Patentamt
European Patent Office
Office européen des brevets

(11) Veröffentlichungsnummer: **0 149 201**
**B1**

(12) **EUROPÄISCHE PATENTSCHRIFT**

(45) Veröffentlichungstag der Patentschrift: **25.02.87**

(21) Anmeldenummer: **84116010.4**

(22) Anmeldetag: **20.12.84**

(51) Int. Cl.⁴: **C 07 C 149/243**, C 07 C 147/14, C 07 C 147/02, A 61 K 31/19, A 61 K 31/22

(54) **Neue Carbonsäure-Derivate, Verfahren zu ihrer Herstellung sowie diese Verbindungen enthaltende Arzneimittel.**

(30) Priorität: **10.01.84 DE 3400514**

(43) Veröffentlichungstag der Anmeldung: **24.07.85 Patentblatt 85/30**

(45) Bekanntmachung des Hinweises auf die Patenterteilung: **25.02.87 Patentblatt 87/9**

(84) Benannte Vertragsstaaten: **AT BE CH DE FR GB IT LI LU NL SE**

(56) Entgegenhaltungen:
**EP-A-0 021 637**
**DE-A-3 007 398**
**GB-A-606 188**
**US-A-3 770 822**

(73) Patentinhaber: **Boehringer Mannheim GmbH, Sandhofer Strasse 116, D-6800 Mannheim 31 (DE)**

(72) Erfinder: **Eggerer, Hermann. Prof.Dr., Egenhofenstrasse 21 B, D-8033 Planegg (DE)**
Erfinder: **Hagenbruch, Bernd. Dr., In den Gärten 5, D-6840 Lampertheim (DE)**
Erfinder: **Nguyen. Tran Giac. Dr., Landhoffstrasse 5, D-8000 München 60 (DE)**
Erfinder: **Stegmeier, Karlheinz, Dr., Kirchbergstrasse 17, D-6148 Heppenheim (DE)**
Erfinder: **Pill, Johannes, Dr.rer.nat.Dr.med., In der Keitgasse 5, D-6906 Leimen 3 (DE)**

**EP 0 149 201 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Vorliegende Erfindung betrifft neue Carbonsäure-Derivate der allgemeinen Formel I

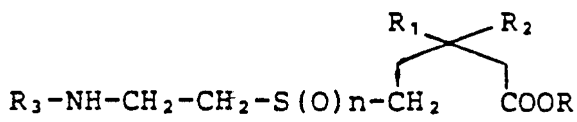

I

in der
R H, Alkyl, ein Metall-Kation, Ammonium, Alkylammonium
$R_1$ H, OH, Alkyl, O-Alkyl, O-Benzyl, O-Acyl
$R_2$ H, Alkyl, Aryl, Aralkyl
n 0, 1, 2
$R_3$ Acyl, wobei dieser Rest
a) eine geradkettige oder verzweigte, gesättigte oder ungesättigte Alkanoylgruppe mit 2 - 11 C-Atomen oder
b) einen Rest

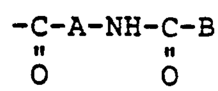

in dem A eine geradkettige oder verzweigte, gesättigte oder ungesättigte Alkylenkette mit 1 - 4 C-Atomen, die gegebenenfalls durch Hydroxy substituiert ist, und
B eine geradkettige oder verzweigte, gesättigte oder ungesättigte Alkylgruppe mit 1 - 8 C-Atomen, die gegebenenfalls ein- oder mehrfach durch Hydroxy, Carboxyl oder Phenyl substituiert ist, Phenyl oder Cycloalkyl oder
c) einen Rest

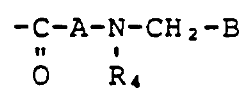

in dem A und B dieselben Gruppen wie unter b) und
$R_4$ H, Alkyl oder Aralkyl darstellen,
bedeutet, sowie deren pharmakologisch verträgliche Salze.

Weiterer Gegenstand der Erfindung sind Verfahren zur Herstellung der Verbindungen der allgemeinen Formel I sowie diese Verbindungen enthaltende Arzneimittel.

Unter der Alkylgruppe der Substituenten R, $R_1$, $R_2$ und $R_4$ werden geradkettige oder verzweigte Reste mit 1 - 4 C-Atomen verstanden. In diesem Sinne bevorzugt sind der Methyl- und Ethylrest.

Entsprechendes gilt für O-Alkyl-Substituenten. Für den Fall, daß $R_1$ = O-Acyl ist, bedeutet Acyl den

Säurerest einer aliphatischen oder araliphatischen Carbonsäure, wobei bevorzugt sind: Acetyl, Propionyl, Butyryl.

Wenn $R_2$ die Bedeutung Aryl hat, soll darunter bevorzugt der Phenylrest verstanden werden, der gegebenenfalls substituiert sein kann, z.B. durch Halogen oder Methoxy.

Gesättigte Alkanoylgruppen des Substituenten $R_3$ sind bevorzugt Acetyl, Propionyl und Heptanoyl; ungesättigte insbesondere Propenoyl.

Unter Cycloalkyl des Restes B sind Reste mit 3-7 C-Atome zu verstehen, insbesondere der Cyclopropyl-, Cyclopentyl und Cyclohexyl-Rest.

Unter der Aralkylgruppe des Substituenten $R_2$ bzw. $R_4$ sind bevorzugt zu verstehen: Benzyl und Phenethyl.

Das in der Beschreibung des Restes $R_3$ verwendete A bedeutet bevorzugt

$-CH_2-$, $-(CH_2)_2-$, $-(CH_2)_3-$ sowie

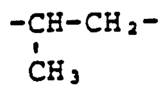,
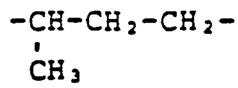

B bedeutet bevorzugt

$-CH_3$ $-CH_2-CH_3$, $-CH_2-CH_2-CH_3$ sowie

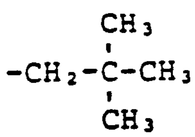,
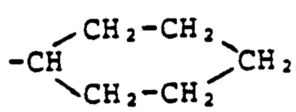,
oder

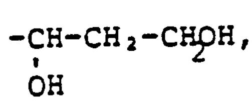,
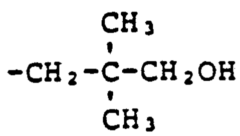,
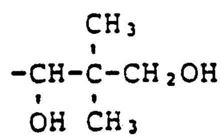
und

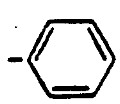

$R_4$ bedeutet bevorzugt. Methyl, Ethyl, Propyl oder Benzyl.

Die obige Definition der erfindungsgemäßen Verbindungen soll auch alle möglichen Stereoisomeren sowie ihre Mischungen umfassen.

Aus der Literatur sind einige, die Teilstruktur Coenzym A enthaltende Thioether bekannt, die als Substrat oder Inhibitor von bestimmten Enzymen fungieren.

So übt die Substanz 3,4-Dicarboxy-3-hydroxy-butyl-Coenzym A eine Hemmwirkung. auf das Enzym Citrat-Synthase aus (Angew. Chem. 92, 133 (1980)).

Ein strukturell ähnlicher Thioether, nämlich 3-Oxobutyl-Coenzym A, wird als. Substrat der HMG-CoA-Synthase beschrieben (J.Biol.Chem. 257, 2842 (1982)).

Weiter ist bekannt, daß 3-Hydroxy-3-methyl-4-carboxybutyl-Coenzym A eine inhibierende Wirkung auf das Enzym HMG-CoA-Reduktase besitzt (FEBS Letters 128, 145 (1981)).

Es wurde nun gefunden, daß schwefelsubstituierte Pentancarbonsäuren der allgemeinen Formel I, strukturell

also wesentlich einfacher als die obigen Verbindungen, ebenfalls eine Hemmung der HMG-CoA-Reduktase bewirken und darüber hinaus gute lipidsenkende Eigenschaften besitzen.

Die Carbonsäuren der allgemeinen Formel I können hergestellt werden, indem man in an sich bekannter Weise

aa) eine Verbindung der Formel II

$R_3$-NH-$CH_2$-$CH_2$-X

mit einer Verbindung der Formel III

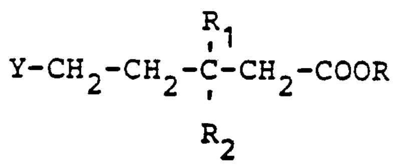

III

umsetzt,

wobei R, $R_1$, $R_2$ und $R_3$ die angegebene Bedeutung haben und X und Y einen reaktiven Rest darstellen, mit der Maßgabe, daß einer der beiden Reste eine -SH-Gruppe sein muß, oder

bb) eine Verbindung der Formel II

$R_3$-NH-$CH_2$-$CH_2$-X

mit einer Verbindung der Formel IV

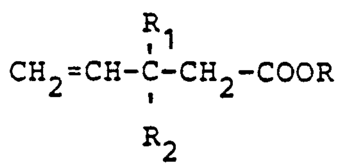

IV

umsetzt,

wobei R, $R_1$, $R_2$ und $R_3$ die angegebene Bedeutung haben und X eine SH-Gruppe darstellt, oder

cc) eine Verbindung der Formel V

$R_3$-Z    V

mit einer Verbindung der Formel VI

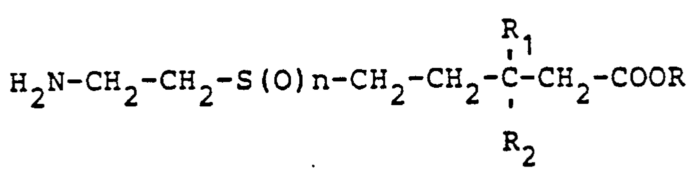

VI

umsetzt,

wobei R, $R_1$, $R_2$, $R_3$ und n die angegebene Bedeutung haben und Z eine reaktive Gruppe darstellt,

anschließend gewünschtenfalls Verbindungen der Formel I, in denen n = 0 oder 1 bedeutet, zu Sulfoxiden oder Sulfonen oxidiert, sowie die erhaltenen Verbindungen gewünschtenfalls in ihre pharmakologisch unbedenkliche Salze überführt.

Im Verfahren aa) bedeuten X und y reaktive Reste, wobei entweder X oder Y eine Thiol-Gruppe sein soll, der jeweils andere Rest aber eine leicht substituierbare Gruppe wie etwa Halogenid, insbesondere Chlorid und Bromid, oder aber ein geeigneter Sulfonsäureester, z.B. Mesylat oder Tosylat. Außerdem kann X bzw. Y auch eine phosphororganische Estergruppe sein, wie z.B. $-O-P(O)Ar_2$ (vgl. Syntheses 1974, 818).

Die Umsetzungen der Verbindungen II und III erfolgen zweckmäßigerweise unter Zusatz eines säurebindenden Mittels, wie z.B. Natriumcarbonat, Kaliumcarbonat. Aber auch stärker basische Agenzien kommen zur Anwendung, wie z.B. Natriumhydroxid, Kaliumhydroxid, Bariumhydroxid sowie Alkoholate wie Natriummethylat, -ethylat, Kalium-tert.-butylat und gegebenenfalls Natriumhydrid.

Als Lösungsmittel kommen vor allem dipolar protische oder aprotische in Frage, z.B. Methanol, Ethanol, Propanol, Wasser, Aceton, Butanon-2, Dimethylformamid, Dimethylsulfoxid sowie Gemische derselben, daneben aber auch unpolar aprotische Lösungsmittel wie Toluol, Tetrahydrofuran.

Weiterhin sind auch Umsetzungen in mehrphasigen Gemischen, z.B. Wasser/Toluol bei Anwesenheit eines basischen Agenz wie Natrium-, Kaliumhydroxid, Kaliumcarbonat, unter Zusatz eines sogenannten Phasentransfer-Katalysators, z.B. Tetraalkylammoniumsalz, möglich.

Es versteht sich, das bestimmte, weitere im Molekül enthaltene, gegen diese Methoden empfindliche Gruppen wie Hydroxy, Amino, Carboxyl in geeigneter Weise intermediär geschützt werden, wobei sich dem Alkylierungsschritt eine Abspaltung der Schutzgruppe anschließt.

Die Addition von Verbindungen der allgemeinen Formel II (X = SH) an die Olefine IV erfolgt in gewünschter Weise in Gegenwart von Radikalkettenstartern wie Sauerstoff, Peroxiden, Azonitrilen, Metalloxiden, Metallsalzen und bei zusätzlicher oder alleiniger Einwirkung von UV-Strahlen (vgl. Org.React. 13, 150). Dabei kann ohne Lösungsmittel oder aber in allen gängigen inerten organischen Lösungsmitteln gearbeitet werden. Die Reaktionstemperatur wird im wesentlichen von den verwendeten Radikalstartern bestimmt, falls diese thermisch gespalten werden sollen.

Die Umsetzung gemäß Verfahren cc) erfolgt durch Reaktion der Carbonsäurederivate V mit den Aminkomponenten VI, wobei diese als freie Base oder deren Salz eingesetzt werden können. Als reaktive Derivate der Carbonsäuren finden vor allem Säurechloride Verwendung, ebenso gut können aber auch deren Ester, Azide, Anhydride und gemischten Anhydride eingesetzt werden.

Die Reaktion wird zweckmäßigerweise unter wasserfreien Bedingungen durchgeführt in inerten Lösungsmitteln wie z.B. Dichlormethan, Tetrahydrofuran, bisweilen aber auch in Wasser oder in Zweiphasensystemen. Als säurebindende Mittel dienen ünorganische oder organische basische Verbindungen, vorzugsweise Pyridin, Triethylamin, Dimethylaminopyridin, Ethyldiisopropylamin aber auch Natriumcarbonat, Natriumhydroxid.

Ausgehend von den Sulfiden I (n = 0) werden die Sulfoxide I (n = 1) durch Umsetzung mit üblichen Oxidationsmitteln erhalten, wie z.B. Natriumperjodat (vgl. J.Org.Chem. 27, 282), Wasserstoffperoxid bei niedriger Temperatur, Natriumdichromat, Distickstofftetroxid, Dimethylsulfoxid, Sauerstoff, tert. Butylhypochlorit. Als dafür geeignete Lösungsmittel finden nach Löslichkeit des Oxidationsmittels Anwendung: Wasser, Aceton, Essigsäure, aliphatische Alkohole, Benzol, Ether.

Zur Herstellung der Sulfone werden Oxidationsmittel wie Wasserstoffperoxid bei erhöhter Temperatur, Persäuren, Kaliumpermanganat, Chromsäure eingesetzt, wobei man zweckmäßigerweise vom Sulfid I (n = 0) ausgeht, gegebenenfalls aber auch von Sulfoxid I (n = 1). Die Wahl der Reaktionsbedingungen und des Reagenz hängt von anderen im Molekül befindlichen, oxidationsempfindlichen Gruppen wie Hydroxy, Amino ab, die gegebenenfalls in bekannter Weise vorübergehend geschützt werden müssen.

Die ebenfalls beanspruchten Ester der Formel I (R = Alkyl) treten entweder als Vorstufen auf, aus denen durch Verseifung mit Mineralsäure oder Alkalihydroxiden in einem polaren Lösungsmittel (wie Wasser, Methanol, Ethanol, Dioxan, Aceton) die freien Carbonsäuren ( R = H) erhalten werden.

Vorteilhaft wird die Verseifung mit einer starken Base (wie Natrium- oder Kaliumhydroxid) in einem Gemisch aus Methanol und Wasser bei Raumtemperatur oder bei mäßig erhöhten Temperaturen durchgeführt. Umgekehrt kann man aber auch die Carbonsäuren in üblicher Weise verestern oder Ester mit einem bestimmten Rest R durch Umestern in einen Ester mit einem anderen Rest R umwandeln. Die Veresterung der Carbonsäuren wird zweckmäßig in Gegenwart eines sauren Katalysators, wie z.B. Chlorwasserstoff, Schwefelsäure, p-Toluolsulfonsäure oder eines stark sauren Ionenaustauschharzes, vorgenommen. Umesterungen hingegen erfordern den Zusatz einer geringen Menge einer basischen Substanz, z.B. eines Alkali- oder Erdalkalihydroxids oder eines Alkalialkoholats. Für die Veresterung der Carboxylgruppe bzw. für eine Umesterung eignen sich prinzipiell alle Alkohole. Bevorzugt sind die niederen einwertigen Alkohole wie Methanol, Ethanol oder Propanol, sowie mehrwertige Alkohole, z.B. Glycerin, oder Alkohole mit anderen funktionellen Gruppen , wie Ethanolamin oder Glykolether.

Zur Herstellung von Salzen mit pharmakologisch verträglichen organischen oder anorganischen Basen, wie z.B. Natriumhydroxid, Kaliumhydroxid, Calciumhydroxid, Ammoniumhydroxid, Methylglukamin, Morpholin oder Ethanolamin können die Carbonsäuren mit den entsprechenden Basen umgesetzt werden. Auch Mischungen der Carbonsäuren mit einem geeigneten Alkalicarbonat bzw. -hydrogencarbonat kommen in Betracht.

Zur Herstellung der Verbindungen II geht man von den reaktiven Carbonsäurederivaten V aus und setzt diese in bekannter Weise mit geeigneten Aminen um, z.B. Aziridin, 2-Aminoethanol, 2-Aminoethylbromid, wobei gegebenenfalls durch eine Folgereaktion die reaktive Gruppe X erzeugt werden muß, z.B. aus Hydroxy durch Austausch gegen Halogen oder Umwandlung in Sulfonsäureester.

Die Amidbildung erfolgt in Gegenwart von anorganischen oder organischen säurebindenden Mitteln, wie z.B. Natriumcarbonat, Triethylamin, vorzugsweise in inerten Lösungsmitteln.

Für den Fall X = SH setzt man die Verbindungen V in der oben beschriebenen Weise mit Cystamin (oder Cystamindihydrochlorid) um und spaltet das Disulfid reduktiv z.B. mit Alkalimetall/ Ammoniak, Metallen (Zink, Aluminium, Eisen, Zinn) in Säuren (Essigsäure, Salzsäure, Schwefelsäure), Alkali-Amalgamen, oder aber mittels Alkalisulfiden oder Alkali-tetrathionaten.

Eine anderew Möglichkeit besteht im Austausch der reaktiven Gruppe X gegen SH, wobei hier eine direkte Umsetzung mit Alkalihydrogensulfiden oder aber schwefelhaltigen Kohlensäurederivaten (wie z.B. Xanthogenat, Dithiourethan, Isothioroniumsalz) oder aber Alkalithiosulfaten erfolgen kann. Bei Anwendung der beiden letzteren Methoden schließt sich eine hydrolytische Spaltung zum Thiol an, die sauer oder alkalisch erfolgen kann.

Die Verbindungen III werden ausgehend von disubstituierten Glutarsäurederivaten (z.B. Anhydriden, Estern, Nitrilen) erhalten, wobei man z.B. das Anhydrid mittels Alkalimetall nach Bouveault-Blanc in die Hydroxysäure oder δ-Lacton überführen kann. Diese Umsetzung ist auch mit komplexen Hydriden möglich, wobei diese auch zur Reduktion des Halbesters eingesetzt werden können.

Überführung der Hydroxysäure bzw. des δ-Lactons in eine in 5-Stellung einen reaktiven Rest tragende Verbindung gelingt z.B. mit Bromwasserstoff/Ethanol, Phosphortribromid, Sulfonsäurechlorid/Base, Benzyltriphenoxyphosphoniumbromid (Beispiel 1), wobei gegebenenfalls diese Methoden behindernde oder gegen diese Methoden empfindliche Gruppen intermediär geschützt werden.

Für die Herstellung von III (Y = SH) werden die vorstehend beschriebenen Verbindungen eingesetzt, die Methoden zur Thiol Darstellung entsprechen im wesentlichen den für II (X = SH) beschriebenen.

Die Zwischenverbindung IV kann aus III (Y ≠ SH) durch β-Eliminierung erhalten werden, daneben ist ein Aufbau mittels Claisen-Umlagerung möglich (vgl. J.Org.Chem. 41, 885), oder aber eine Hydrolyse des entsprechenden Dichloralkens möglich (vgl. CA 93, 94833 u)

$$Cl-CH_2-CH_2-\overset{R_1}{\underset{R_2}{C}}-CH=CCl_2 \longrightarrow\;\longrightarrow IV$$

Die im Verfahren cc) eingesetzten Verhindungen VI sind in üblicher Weise aus III (Y = SH) durch Umsetzung mit Aziridin oder aus III (Y ≠ SH) durch Umsetzung mit Cysteamin bzw. einem eine geeignete Aminschutzgruppe tragenden 2-Aminoethyl-thiol zugänglich (n = 0), wobei gegebenenfalls anschließend zu Sulfoxiden und Sulfonen oxidiert wird.

Zur Herstellung von Arzneimitteln werden die Verbindungen der allgemeinen Formel I in an sich bekannter Weise mit geeigneten pharmazeutischen Trägersubstanzen, Aroma-, Geschmacks- und Farbstoffen gemischt und beispielsweise als Tabletten oder Dragees ausgeformt oder unter Zugabe entsprechender Hilfsstoffe in Wasser oder Öl, wie z.B. Olivenöl, suspendiert oder gelöst.

Die Substanzen der allgemeinen Formel I können in flüssiger oder fester Form oral und parenteral appliziert werden. Als Injektionsmedium kommt vorzugsweise Wasser zur Anwendung, welches die bei Injektionslösungen üblichen Stabilisierungsmittel, Lösungsvermittler und/oder Puffer enthält. Derartige Zusätze sind z.B. Tartrat- oder Borat-Puffer, Ethanol, Dimethylsulfoxid, Komplexbildner (wie Ethylendiamintetraessigsäure), hochmolekulare, Polymere (wie flüssiges Polyethylenoxid) zur Viskositätsregulierung oder Polyethylen-Derivate von Sorbitanhydriden.

Feste Trägerstoffe sind z.B. Stärke, Lactose, Mannit, Methylcellulose, Talkum, hochdisperse Kieselsäure, höhermolekulare Fettsäuren (wie Stearinsäure), Gelatine, Agar-Agar, Calciumphosphat, Magnesiumstearat, tierische und pflanzliche Fette oder feste hochmolekulare Polymere (wie Polyethylenglykole). Für die orale Applikation geeignete Zubereitungen können gewünschtenfalls Geschmacks- und Süßstoffe enthalten.

Die verabreichte Dosierung hängt vom Alter, der Gesundheit und dem Gewicht des Empfängers, dem Ausmaß der Krankheit, der Art gleichzeitiger gegebenenfalls durchgeführter weiterer Behandlungen, der Häufigkeit der Behandlungen und der Art der gewünschten Wirkung ab. Üblicherweise beträgt die tägliche Dosis der aktiven Verbindung 0.1 bis 50 mg/kg Körpergewicht. Normalerweise sind 0.5 bis 40 und vorzugsweise 1.0 bis 20 mg/kg/Tag in einer oder mehreren Anwendungen pro Tag wirksam, um die gewünschten Resultate zu erzielen.

Bevorzugt im Sinne der Erfindung sind außer den in den Beispielen genannten Verbindungen die folgenden

Verbindungen der Formel I sowie deren Methyl- oder Ethylester und Natrium-, Kalium-, Calcium- oder Ammonium-Salze:

1. 9,13-Aza-3,3-dimethyl-10,14-oxo-6-thi-pentadecansäure
2. 9,13-Aza-15,17-dihydroxy-3,3-dimethyl-10,14-oxo-6-thia-heptadecansäure
3. 9,13-Aza-17-hydroxy-10,14-oxo-3,3,16,16-tetramethyl-6-thia-heptadecansäure
4. 9,13-Aza-10-oxo-3,3,16,16-tetramethyl-6-thia-heptadecansäure
5. 9,13-Aza-13-butyl-3,3-dimethyl-10-oxo-6-thia-heptadecansäure
6. 9,13-Aza-13-benzyl-3-ethyl-3-methyl-10-oxo-6-thia-pentadecansäure.
7. 9,13-Aza-15,17-dihydroxy-10-oxo-6-thia-heptadecansäure
8. 9,13-Aza-17-hydroxy-10-oxo-3,3,16,16-tetramethyl-6-thia-heptadecansäure
9. 9,13-Aza-3-methoxy-3-methyl-10-oxo-14-phenyl-6-thia-tetradecansäure
10. 9,13-Aza-15,17-dihydroxy-3-methoxy-10,14-oxo-6-thia-3,16,16-trimethyl-heptadecansäure
11. 9,13-Aza-14-cyclohexyl-3-methyl-10,14-oxo-3-phenyl-6-thia-tetradecancarbonsäure

11a) 9,13-Aza-16,16-dimethyl-10,14-oxo-6-thia-heptadecansäure

12. 9,13-Aza-6,10,14-oxo-3,3,16,16-tetramethyl-6-thia-heptadecansäure
13. 9,13-Aza-3,3-dimethyl-6,10,14-oxo-14-phenyl-6-thia-tetradecansäure
14. 9,13-Aza-16,16-dimethyl-6,6,10,14-oxo-6-thia-heptadecansäure
15. 9,13-Aza-3,14-diphenyl-3-methyl-6,6,10,14-oxo-6-thia-tetradecansäure

Erlaeuterung Die hochgestellten Buchstaben bei den $R_F$-Werten stehen fuer bestimmte Laufmittelgemische:
A: Toluol/Ethanol = 1/1
B: Essigsaeure/n-Butanol/Wasser = 15/60/25
C: Butylacetat/Isopropanol/Wasser/$NH_3$ = 30/50/15/5
D: Dichlormethan/Aceton = 1/1
E: Chloroform/2-Butanon/Methanol/Essigsaeure/Wasser = 75/25/35/5/9
Als stationaere Phase diente Kieselgel.

**Beispiel 1**

**5-Bromo-3-hydroxy-3-methyl-pentasäuremethylester**

Zu einer Lösung von 3.00 g (18.5 mmol) 3,5-Dihydroxy-3-methyl-pentasäuremethylester in 15 ml wasserfreiem Tetrahydrofuran gibt man eine Lösung von 8.90 g (18.5 mmol) Benzyltriphenoxyphosphoniumbromid in 10 ml Tetrahydrofuran und rührt 15 h bei Raumtemperatur. Danach engt man im Vakuum ein, wobei die Badtemperatur unter 15°C gehalten wird. Der ölige Rückstand wird an 700 g Kieselgel 60 mit n-Hexan/t-Butanol (6:1) als Laufmittel chromatographiert.

Nach Entfernen des Laufmittels erhält man 1.32 g (32 %) als hellgelbes Öl,
$n^{20}_D{}'$ = 1.4734, $R_f$ = 0.65 (Kieselgel; n-Hexan/t-Butanol (6:1)). IR (Film): 3500 (OH), 2950-2970 (CH), 1725 (C=O),
1200 (C-O), 650 $cm^{-1}$ (C-Br)
$^1$H-NMR ($CCl_4$): δ = 1.2 (s; 3 H), 2.0 (t, J = 3 Hz; 2 H),
2,4 (s; 2 H), 3.4 (t, J = 3 Hz; 2 H), 3.7 (s; 3 H)

**Beispiel 2**

**9,13-Aza-10,14-oxo-6-thia-3,15,17-trihydroxy-3,16,16-trimethyl-heptadecansäure**

Man rührt eine Lösung von 17.8 mg (0,10 mmol) 3,7-Aza-9,11-dihydroxy-10,10-dimethyl-4,8-oxo-undecanthiol und 125 mg (0,55 mmol) 5-Bromo-3-hydroxy-3-methyl-pentan-säuremethylester in 10 ml 50 proz. methanolischer 0.25 M Natriumcarbonat - 0.1 N Natronlauge unter Stickstoffatmosphäre 5 h. Man engt ein und reinigt durch Säulenchromatographie an DEAE-Sepharose mit Lithiumchlorid-Gradient Eluation (linear; 0-0.2 molar).

Man erhält 20 mg (49 %) sehr hygroskopischen Feststoff:
$R_f$ = 0.49 (Kieselgel; Essigsäure /n-Butanol/Wasser = 15/60/25).

**Beispiel 3**

**9-Aza-3-hydroxy-3-methyl-10-oxo-6-thia-heptadecansäure**

Eine Lösung von 406 mg (2.00 mmol) N-Octanoyl-cysteamin und 426 mg (2.10 mmol) 5-Brom-3-hydroxy-3-methyl-pentansäure in 50 ml 50 proz. methanolischer 0.2 N Natronlauge wird unter Stickstoffatmosphäre 8 h gerührt, angesäuert und mit Essigester extrahiert. Der nach dem Trocknen ($Na_2SO_4$) und Einengen der organischen Phase erhaltene Rückstand wird mehrmals aus Methanol/Ether umkristallisiert.

275 mg (40 %), vom Schmp. : 99°C.
$R_f$ = 0.72 (Kieselgel; Essigsäure/n-Butanol/Wasser = 15/60/25).

**Beispiel 4**

**5-Bromo-3-methyl-pentansaeureethylester**

Man saettigt eine Loesung von 16.0 g (0.14 mol) 4-Methyl-3,4,-5,6-tetrahydro-2-pyranon in 50 ml wasserfreiem Ethanol bei 0 - 5°C mit HBr-Gas, laeßt ueber Nacht stehen, gießt auf Wasser und extrahiert mit Ether. Die vereinigten Etherextrakte werden ueber $Na_2SO_4$ getrocknet und eingeengt, der Rueckstand im Vakuum destilliert: 21.9 g (70 %) farblose Fluessigkeit vom Sdp.: 85-7°C (0.1 mm). $n^{20}_D$ = 1 4590.

$^1$H-NMR ($CDCl_3$): δ = 0.98 (d, J= 6 Hz;, 3H), 1.29 (t, J = 7 Hz; 3H), 1.67-2.16 (mc; 3H), 2.25 (s, breit; 2H), 3.45 (t, J = 7 Hz; 2H), 4.15 (g, J = 7 Hz; 2H).

In Analogie dazu stellt man folgende Verbindungen her:
4 a) aus 4-Ethyl-4-methyl-3,4,5,6-tetrahydro-2-pyranon und Methanol
5-Bromo-3-ethyl-3-methyl-pentansaeuremethylester
Ausbeute: 75 %; Sdp.: 56-8°C (0.01 mm);
$n^{20}_D$= 1.4608.
$^1$H-NMR ($CDCl_3$): d = 0.85 (t, J = 7 Hz; 3H), 1.00 (s; 3H), 1.37 (q, J = 7 Hz; 2H), 1.73-2.13 (m; 2H), 2.2 (s; 3H), 3.22-3.58 (m; 2H), 3.65 (s; 3H).
4 b) aus 4-Phenyl-3,4,5,6-tetrahydro-2-pyranon und Ethanol
5-Bromo-3-phenyl-pentansaeureethylester
Ausbeute: 89 %; Sdp.: 126-31°C (0.1 mm);
$n^{20}_D$ = 1.5216.
$^1$H-NMR ($CDCl_3$): δ= 1.13 (t, J = 7 Hz; 3H), 2.05-2.40 (t, J = 6.5 Hz; 2H), 2.61 (d, J = 7.5 Hz; 2H), 2.97-3.55 (m; 3H), 4.06 (q, J = 7 Hz; 2H), 7.27 (s; 5H).
4 c) aus 4-Methyl-4-phenyl-3,4,5,6-tetrahydro-2-pyranon und Ethanol
5-Bromo-3-methyl-3-phenyl-pentansaeureethylester
Ausbeute: 80 %; Sdp.-. 151-5°C (0.1 mm);
$n^{20}_D$ = 1.5289.
$^1$H-NMR ($CDCl_3$): δ = 1.08 (t, J = 7 Hz, 3H), 1.52 (s, 3H), 2.00-2.55 (m; 2H), 2.64 (s, 2H), 2.82-3.34 (m; 2H), 4.00 (q, J = 7 Hz; 2H), 7.3 (s; 5H).
4 d) aus 5,6-Dihydro-2-pyranon und Ethanol
5-Bromo-3-ethoxy-pentansäureethylester
Ausbeute: 23 %; Sdp.: 64°C/0.02 mm
$n^{20}_D$ = 1.4560.
$^1$H-NMR ($CDCl_3$): δ = 1.18 (t, J = 7 Hz; 3H), 1.26 (t, J = 7 Hz; 3H), 2.00-2.14 (m; 2H), 2.38-2.66 (m; 2H), 3.42-3.66 (m; 4H), 3.86-3.96 (m; 1H), 4.14 (q , J = 7 Hz; 2H).

**Beispiel 5**

**3.7-Aza-71tert.-butoxycarbonyl)-4-oxo-8-phenyl-octanthiol**

Zu einer Loesung von 5.61 g (20.0 mmol) 4-Aza-4-(tert.-butoxycarbonyl)-5-phenyl-pentansaeure in 60 ml wasserfreiem $CH_2Cl_2$ und 2.02 g (20.0 mmol) Triethylamin gibt man innerhalb 30 min bei -10°C 2.73 g (20.0 mmol) Chlorameisensaeure-isobutylester, ruehrt 30 min nach, und tropft ebenfalls bei -10°C eine Loesung von 2.27 g (20.0 mmol) 2-Amino-ethanthiol in 60 ml $CH_2Cl_2$ zu. Anschließend laeßt man innerhalb 1 h auf Raumtemperatur kommen, saugt ab und waescht das Filtrat mit Wasser und $NaHCO_3$-Loesung. Man trocknet die organische Phase ueber $Na_2SO_4$ und engt ein: 6.3 g (94 %) farbloses Oel, wird ohne Reinigung weiter umgesetzt;
$R_F^B$ = 0.92; $R_F^C$ = 0.88.
In Analogie dazu erhaelt man mit THF als Loesungsmittel aus
4-Aza-7,7-dimethyl-6-hydroxy-9-oxa-5-oxo-10,10,10-triphenyl-decansaeure
5 a) 3,7-Aza-10,10-dimethyl-9-hydroxy-12-oxa-4,8-oxo-13,13-13-triphenyl-tridecanthiol
Ausbeute: 45 %; Schmp. 63°C (Zers.).
Aus 4-Aza-4-methyl-5-cyclohexyl-pentansaeure
5 b) 3,7-Aza-8-cyclohexyl-7-methyl-4-oxo-octanthiol
Ausbeute: 39 %; als Oxalat gefaellt;
Schmp. 119-21°C (Ethanol).
Aus 4-Aza-4-(tert.-butoxycarbonyl)-6,6-dimethyl-heptansaeure
5 c) 3,7-Aza-7-(tert.-butoxycarbonyl)-9,9-dimethyl-4-oxo-decanthiol
Ausbeute: 90 %; Oel;
$R_F^A$ = 0.89; $R_F^B$ = 0.83.
Aus 4-Aza-4-butyl-octansäure mit $CH_2Cl_2$ als Lösungsmittel

5 d) 3,7-Aza-7-butyl-4-oxo-undecanthiol
Ausbeute: 49 %; als Oxalat gefällt;
Schmp.: 106-7°C (Isopropanol)
$R_F^B$ = 0.25 ; $R_F^C$ = 0.90.

**Beispiel 6**

**3,7-Aza-6-methyl-4,8-oxo-8-phenyl-octanthiol**

In eine geruehrte Mischung aus 5.84 g (11.0 mmol) Bis(3,7-Aza-6-methyl-4,8-oxo-8-phenyl-octan)disulfid, 50 ml 4 N $H_2SO_4$ und 20 ml Ethanol traegt man innerhalb 2 h 7.80 g (0.12 g-Atom) Zinkstaub, saugt ab und extrahiert mit Ether. Die organische Phase wird getrocknet ($Na_2SO_4$) und eingeengt, der erhaltene Rueckstand aus Ethanol umkristallisiert:. 3.5 g (58 %); Schmp. 156-7°C.

In Analogie dazu erhaelt man aus den entsprechenden Disulfiden:

6 a) 3,7-Aza-4,8-oxo-undecanthiol
Ausbeute: 73 %; Schmp. 169-70°C (mit Ligroin ausgeruehrt).

6 b) 3-Aza-4-oxo-hexanthiol
Ausbeute:. 85 %; Oel;,
$R_F^A$ = 0.54; $R_F^B$ = 0.64.

6 c) 3,7-Aza-4,8-oxo-8-phenyl-octanthiol
Ausbeute: 73 %; Schmp. 127-8°C ($CH_2Cl_2$);
wurde kontinuierlich mit $CH_2Cl_2$ extrahiert.

6 d) 3,7-Aza-8-cyclohexyl-4,8-oxo-octanthiol
Ausbeute: 56 %; Schmp. 118°C (Ligroin/Ether)

6 e) 3,7-Aza-10,10-dimethyl-4,8-oxo-undecanthiol
Ausbeute: 44 %; Wachs;,
$R_F^A$ = 0.31

In den Beispielen 6 c bis 6 e wurde bei sonst gleichen Reaktionsbedingungen 6 N HCl statt 4 N $H_2SO_4$ verwendet.

**Beispiel 7**

**9,13-Aza-3,3-dimethyl-10,14-oxo-6-thia-heptadecansaeure-ethylester**

Zu einer geruehrten Loesung von Natriumethylat (aus 0.46 g [20.0 mg-Atom] Natrium) in 50 ml wasserfreiem Ethanol gibt man unter $N_2$-Atmosphaere 4.37 g (20.0 mmol) 3,7-Aza-4,8-oxo-undecanthiol, ruehrt 15 min nach, fuegt 5.22 g (22.0 mmol) 5-Brom-3,3-dimethyl-pentansaeureethylester zu und erhitzt 3 h auf 50-60°C. Danach engt man ein, nimmt den Rueckstand in $CH_2Cl_2$ auf, waescht mit Wasser, trocknet die organische Phase ueber $Na_2SO_4$ und engt ein. Der Rueckstand wird aus Essigester umkristallisiert: 5.0 g (67 %); Schmp. 104-5°C;,
$R_F^C$ = 0.80.

In Analogie dazu werden aus 5-Bromo-3,3-dimethyl-pentansaeure-ethylester die folgenden Produkte dargestellt:

7 a) mit 3,7-Aza-10,10-dimethyl-4,8-oxo-undecanthiol:
9,13-Aza-10,14-oxo-3,3,16,16-tetramethyl-6-thia-heptadecansaeureethylester
Ausbeute: 77 %; wachsartige Substanz vom Schmp. 106-115°C (Ether/Ligroin).

7 b) mit 3,7-Aza-4,8-oxo-8-phenyl-octanthiol:
9,13-Aza-3,3-dimethyl-10,14-oxo-14-phenyl-6-thia-tetradecansaeureethylester
Ausbeute: 90 %; zaehes Oel;
$R_F^A$ = 0.65; $R_F^B$ = 0.61

7 c) mit 3,7-Aza-8-cyclohexyl-4,8-oxo-octanthiol:
9,13-Aza-14-cyclohexyl-3,3-dimethyl-10,14-oxo-6-thia-tetradecansaeureethylester
Ausbeute: 50 %; zaehes Wachs;
$R_F^A$ = 0.70; $R_F^B$ = 0.68.

7 d) mit 3,7-Aza-6-methyl-4,8-oxo-8-phenyl-octanthiol:
9,13-Aza-10,14-oxo-14-phenyl-6-thia-3,3,12-trimethyl-tetradecansaeureethylester
Ausbeute: 31 %; Schmp. 102-4°C;
$R_F^C$ = 0.87.

7 e) mit 3,7-Aza-7-(tert.-butoxycarbonyl)-4-oxo-8-phenyl-octanthiol:
9,13-Aza-13-(tert.-butoxycarbonyl)-3,3-dimethyl-10-oxo-14-phenyl-6-thia-tetradecansaeureethylester
Ausbeute: 95 %; Oel;
$R_F^B$ 0.83; $R_F^C$ 0.91; $R_F^E$ 0.95.

**Beispiel 8**

In Analogie zu Beispiel 7 stellt man durch Umsetzen von 5-Bromo-pentansaeureethylester mit den entsprechenden Thiolen folgende Verbindungen her:
8 a) mit 3-Aza-4-oxo-hexanthiol
9-Aza-10-oxo-6-thia-dodecansaeureethylester
Ausbeute: 72 %; Oel; $n^{20}_D = 1.4871$;
$R_F^A = 0.67$; $R_F^B = 0.63$.
8 b) mit 3,7-Aza-10,10-dimethyl-4,8-oxo-undecanthiol
9,13-Aza-16,16-dimethyl-10,14-oxo-6-thia-heptadecan-saeureethylester
Ausbeute: 71 %; Wachs;
$R_F^A = 0.75$; $R_F^B = 0.81$.
8 c) mit 3,7-Aza-6-methyl-4,8-oxo-8-phenyl-octanthiol
9,13-Aza-12-methyl-10,14-oxo-14-phenyl-6-thia-tetra-decansaeureethylester
Ausbeute: 93 %; Schmp. 100-101°C.

**Beispiel 9**

In Analogie zu Beispiel 7 stellt man durch Umsetzung von 5-Bromo-3-phenyl-pentansaeureethylester mit den entsprechenden Thiolen folgende Verbindungen her:
9 a) mit 3,7-Aza-4,8-oxo-octanthiol
9,13-Aza-10,14-oxo-3-phenyl-6-thia-heptadecansaeure-ethylester
Ausbeute: 80 %; Schmp. 77-80°C (Ether/Ligroin);
$R_F^A = 0.64$; $R_F^B = 0.68$.
9 b) mit 3,7-Aza-6-methyl-4,8-oxo-8-phenyl-octanthiol
9,13-Aza-3,14-diphenyl-12-methyl-10,14-oxo-6-thia-tetradecansaeureethylester
Ausbeute: 85 %; Oel.

**Beispiel 10**

In Analogie zum Beispiel 7 erhaelt man aus 3,7-Aza-8-cyclo-hexyl-4,8-oxo-octanthiol und 5-Bromo-3-methyl-3-phenyl-pentansaeureethylester die Verbindung:
10 a) 9,13-Aza-14-cyclohexyl-3-methyl-10,14-oxo-3-phenyl-6-thia-tetradecansaeureethylester
Ausbeute: 88 %; zaehes Oel; $R_F^B = 0.84$; $R_F^C = 0.78$.
aus 3,7-Aza-4,8-oxo-8-phenyl-octanthiol und 5-Bromo-3-methyl-pentansaeureethylester die Verbindung
10 b) 9,13-Aza-3-methyl-10,14-oxo-14-phenyl-6-thia-tetra-decansaeureethylester
Ausbeute: 83 %; Wachs; $R_FsD = 0.45$
aus 3,7-Aza-7-(tert.-butoxycarbonyl)-4-oxo-8-phenyl-ooctanthiol und 5-Bromo-3-ethyl-3-methyl-pentansaeure-methylester
10 c) 9,13-Aza-13-(tert.-butoxycarbonyl)-3-ethyl-3-methyl-10-oxo-14-phenyl-6-thia-tetradecansaeuremethylester
Ausbeute: 75 %; zaehes Oel;
$R_F^B = 0.81$; $R_F^C = 94$; $R_F^E = 0.93$;
aus 3,7-Aza-10,10-dimethyl-9-hydroxy-11-oxa-4,8-oxo-13,13,13-triphenyl-tridecanthiol und 5-Bromo-3-methyl-3-phenyl-pentansaeureethylester
10 d) 9,13-Aza-15-hydroxy-18-oxa-10,14-oxo-3,19,19,19-tetra-phenyl-6-thia-3,16,16-trimethyl-nonadecansaeureethylester
Ausbeute: 64 %; Schmp. 54°C; $R_F^B = 0.85$.

**Beispiel 11**

**9,13-Aza-15,17-dihydroxy-10,14-oxo-3,3,16,16-tetramethyl-6-thia-heptadecansaeureethylester**

Man ruehrt eine Mischung aus 2.78 g (10.0 mmol) 3,7-Aza-9,11-dihydroxy-10,10-dimethyl-4,8-oxo-undecanthiol, 2.37 g (10.0 mmol) 5-Bromo-3,3-dimethyl-pentansaeureethylester und 1.38 g (10.0 mmol) $K_2CO_3$ in 50 ml Ethanol unter $N_2$-Atmosphaere 4 h bei 50°C, setzt Ether zu und saugt ab. Das Filtrat wird eingeengt, der Rueckstand mehrmals mit Ligroin durchgearbeitet:
3.2 g (75 %) zaehes Wachs; $R_F^B = 0.64$; $R_F^E = 0.52$.
In analoger Weise lassen sich aus obigem Thiol folgende Produkte darstellen:
11 a) mit 5-Bromo-3-methyl-pentansaeureethylester:

9,13-Aza-15,17-dihydroxy-10,14-oxo-6-thia-3,16,16-trimethyl-heptadecansaeureethylester
Ausbeute: 96 %;, Wachs;
$R_F^B$ = 0.79; $R_F^E$ = 0.72.
11 b) mit 5-Bromo-3-ethyl-3-methyl-pentansaeuremethylester:
9,13-Aza-15,17-dihydroxy-3-ethyl-10,14-oxo-6-thia-3-16,16-trimethyl-heptadecansaeuremethylester
Ausbeute: 31 %; Wachs;,
$R_F^B$ = 0.80; $R_F^C$ = 0.75; $R_F^E$ = 0.71.
11 c) mit 5-Bromo-3-methyl-3-phenyl-pentansaeureethylester:
9,13-Aza-15,17-dihydroxy-10,14-oxo-3-phenyl-6-thia-3-16,16-trimethyl-heptadecansaeureethylester
Ausbeute: 41 %; zaehes Oel;
$R_F^C$ = 0.71;, $R_F^E$ = 0.86.
>11 d) mit 5-Bromo-pentansaeureethylester:
9,13-Aza-16,16-dimethyl-15,17-dihydroxy-10,14-oxo-6-thia-heptadecansaeureethylester
Ausbeute: 57 %;, zaehes Oel;
$R_F^B$ = 0.63, $R_F^C$ = 0.68; $R_F^E$ = 0.66.
11 e) mit 5-Bromo-3-phenyl-pentansaeureethylester
9,13-Aza-16,16-dimethyl-15,17-dihydroxy-10,14-oxo-3-phenyl-6-thia-heptadecansaeureethylester
Ausbeute: 50 %; zaehes Oel;
$R_F^B$ = 0.83; $R_F^C$ = 0.74; $R_F^E$ = 0.73.
11 f) mit 5-Bromo-3-ethoxy-pentansäureethylester
9.13-Aza-16,16-dimethyl-15,17-dihydroxy-3-ethoxy-10,14-oxo-6-thia-heptadecansäureethylester
Ausbeute: 20 %, zähes Öl, $R^A_F$ = 0.76;
$R_F^C$ = 0.64; $R^E_F$ = 0.84.
sowie durch Umsetzung von 3,7-Aza-7-(tert.-butoxycarbonyl)-9,9-dimethyl-4-oxo-decanthiol mit 5-Bromo-3,3-dimethyl-pentansaeureethylester
11 g) 9,13-Aza-13-(tert.-butoxycarbonyl)-10-oxo-3,3,15,15-tetramethyl-6-thia-hexadecansaeureethylester
Ausbeute: 90 %; Oel;
$R_F^A$ = 0.90; $R_F^B$ = 0.85
und durch Umsetzung von 3,7-Aza-8-cyclohexyl-7-methyl-4-oxo-octanthiol mit 5-Bromo-3,3-dimethyl-pentansaeureethylester
11 h) 9,13-Aza-14-cyclohexyl-10-oxo-6-thia-3,3,13-trimethyl-tetradecansaeureethylester
Ausbeute: 85 %; Oel;,
$R_F^B$ = 0.45; $R_F^E$ = 0.56.
und durch Umsetzung von 3,7-Aza-7-butyl-4-oxo-undecanthiol mit 5-Bromo-3,3-dimethyl-pentansäureethylester
11 i) 9,13-Aza-13-butyl-3,3-dimethyl-10-oxo-6-thia-heptadecansäureethylester
Ausbeute: 40 %; als Oxalat gefällt;
Schmp.: 109-10°C (Essigester);
$R_F^B$ = 0.22; $R_F^E$ = 0.60.

**Beispiel 12**

**9,12-Aza-10-oxo-13-phenyl-6-thia-tridecansaeureethylester**

Zu einer Loesung von 6.34 g (14.0 mmol) 9,12-Aza-12-(tert.-butoxycarbonyl)-10-oxo-13-phenyl-6-thia-tridecansaeureethylester in 100 ml wasserfreiem THF gibt man unter Eiskuehlung 150 ml gesaettigte etherische HCl, ruehrt 1 h bei 0°C, dann 2 h bei Raumtemperatur, setzt Ether zu und saugt den Niederschlag ab: 4.2 g (78%) Hydrochlorid vom Schmp. 165°C; $R_F^B$ = 0.59.

In Analogie dazu erhaelt man:
12 a) 9,13-Aza-3,3-dimethyl-10-oxo-14-phenyl-6-thia-tetra-decansaeureethylester
Ausbeute: 30 %; Wachs;
$R_F^B$ = 0.71; $R_F^C$ = 0.81.
12 b) 9,13-Aza-3-ethyl-3-methyl-10-oxo-14-phenyl-6-thia -tetradecansaeuremethylester
Ausbeute: 40 %; Wachs;
$R_F^B$ = 0.73; $R_F^C$ = 0.86.
12 c) 9,13-Aza-10-oxo-3,3,15,15-tetramethyl-6-thia-hexa-decansaeureethylester.
Als Loesungsmittel wird nur Ether verwendet;
Ausbeute: 87 %,; zaehes Oel;
$R_F^A$ = 0.47; $R_F^B$ = 0.54.

**Beispiel 13**

**8-Amino-6-thia-octansaeureethylester**

Man ruehrt eine Mischung aus 16.2 g (0.10 mol) 5-Thio-pentan-saeureethylester und 8.60 g (0.20 mol) Ethylenimin 4 h bei 40 C, engt ein und destilliert rasch im Vakuum: 8.6 g (42 %) farblose Fluessigkeit vom Schmp. 130-6 C (0.01 mm);

$R_F^C = 0.49$; $R_F^E = 0.39$.

analog erhaelt man aus 3-Phenyl-5-thio-pentansaeureethyl-ester

13 a) 8-Amino-3-phenyl-6-thia-octansaeure-ethylester

Ausbeute:. 94 % (nicht destillierbar);

$R_F^B = 0.53$; $R_F^C = 0.70$.

**Beispiel 14**

In Analogie der in Beispiel 5 beschriebenen "mixed anhydride" Methode erhaelt man die folgenden Thiopentansaeurederivate:

14 a) durch Umsetzung von 4-Aza-7,7-dimethyl-6-hydroxy-9-oxa-5-oxo-10,10,10-triphenyl-decansaeure mit 8-Amino-3-phenyl-6-thia-octansaeureethylester:

9,13-Aza-16,16-dimethyl-15-hydroxy-10,14-oxo-18-oxa-3,19,19,19-tetraphenyl-6-thia-nonadecansaeure-ethylester

Ausbeute: 87 %; wachsartig;

$R_F^B = 0.97$; $R_F^E = 0.86$.

14 b) durch Umsetzung von 3-Aza-3-(tert.-butoxycarbonyl)-4-phenyl-butansaeure mit 8-Amino-6-thia-octansaeureethylester:

9,12-Aza-12-(tert.-butoxycarbonyl)-10-oxo-13-phenyl-6-thia-tridecansaeureethylester

Ausbeute: 90 %; Oel, wird roh weiterverarbeitet.

**Beispiel 15**

**9,13-Aza-3,3-dimethyl-10,14-oxo-6-thia-heptadecansaeure**

Man ruehrt eine Loesung von 3.93 g (10.5 mmol) 9,13-Aza-3,3-dimethyl-10,14-oxo-6-thia-heptadecansaeureethylester in 21 ml 1 N KOH und 10 ml Ethanol 2 h bei 60°C, destilliert das Ethanol ab, extrahiert mit $CH_2Cl_2$ und saeuert mit 2 N HCl an. Die waessrige Phase wird mehrmals mit $CH_2Cl_2$ extrahiert, die organische Phase ueber $Na_2SO_4$ getrocknet und eingeengt. Der Rueckstand wird durch Verreiben mit Ligroin zur Kristallisation gebracht: 2.6 g (71 %); Schmp. 72-3°C;,

$R_F^C = 0.35$.

In analoger Weise werden aus den entsprechenden 5-Thiopentan-saeureestern folgende Carbonsaeuren hergestellt:

15 a) 9,13-Aza-10,14-oxo-3,3,16,16-tetramethyl-6-thia-heptadecansaeure

Ausbeute: 53 %; Schmp. 61-5°C (Ligroin/Ether);

$R_F^A = 0.53$, $R_F^B = 0.81$ .

15 b) 9,13-Aza-3,3-dimethyl-10,14-oxo-14-phenyl-6-thia-tetradecansaeure

Ausbeute: 19 %; Schmp. 75-7°C (Essigester);

$R_F^A = 0.55$; $R_F^B = 0.81$.

15 c) 9,13-Aza-14-cyclohexyl-3,3-dimethyl-10,14-oxo-6-thia-tetradecansaeure

Ausbeute: 21 %; Schmp. 123 C;

$R_F^A = 0.68$; $R_F^B = 0.78$ .

15 d) 9,13-Aza-10,14-oxo-14-phenyl-6-thia-3,3,12-trimethyl-tetradecansaeure

Ausbeute: 30 %; Schmp. 111-2 C;

$R_F^C = 0.36$.

15 e) 9-Aza-10-oxo-6-thia-dodecansaeure

Ausbeute: 62 %; Schmp. 45-7 C (Ligroin/Ether);

$R_F^A = 0.54$; -$R_F^B = 0.64$.

15 f) 9,13-Aza-16,16-dimethyl-10,14-oxo-6-thia-heptadecansaeure

Ausbeute: 76 %; Schmp. 74-7°C (Ether);

$R_F^A = 0.46$; $R_F^B = 0.72$.

15 g) 9,13-Aza-12-methyl-10,14-oxo-14-phenyl-6-thia-tetradecansaeure

Ausbeute:. 33 %;, Schmp. 102-4°C;

$R_F^B = 0.85$; $R_F^C = 0.37$.

15 h) 9,13-Aza-10,14-oxo-3-phenyl-6-thia-heptadecansaeure

Ausbeute.: 62 %;, Schmp. 84-8 C (Ether/Ligroin);

$R_F^A = 0.47$; $R_F^B = 0.71$.

15 j) 9,13-Aza-3,14-diphenyl-12-methyl-10,14-oxo-6-thia-tetradecansaeure
Ausbeute: 40 %; Schmp. 63-6 C (Ether/Ligroin);
$R_F^C = 0.38$.

15 k) 9,13-Aza-14-cyclohexyl-3-methyl-10,14-oxo-3-phenyl-6-thia-tetradecansaeure
Ausbeute: 43 %; Schmp. 109-11 C (Ether);
$R_F^A = 0.57$; $R_F^B = 0.83$.

15 l) 9,13-Aza-3-methyl-10,14-oxo-14-phenyl-6-thia-tetra-decansaeure
Ausbeute: 78 %; Schm. 69-70 C (Ether/Ligroin)
$R_F^B = 0.80$; $R_F^C = 0.38$.

15 m) 9,13-Aza-3,3-dimethyl-10-oxo-14-phenyl-6-thia-tetra-decansaeure
Ausbeute: 38 %; Schmp. 97-99 C; $R_F^B = 0.70$; $R_F^E = 0.58$.

15 n) 9,13-Aza-3-ethyl-3-methyl-14-phenyl-10-oxo-6-thia-tetradecansaeure
Ausbeute: 37 %; Schmp. 94-7 C;
$R_F^B = 0.71$; $R_F^E = 0.61$.

15 o) 9,13-Aza-15,17-dihydroxy-10,14-oxo-3,3,16,16-tetra-methyl-6-thia-heptadecansaeure
Ausbeute: 25 %; Schmp. 73-83 C;
$R_F^B = 0.79$; $R_F^C = 0.34$; $R_F^E = 0.61$.

15 p) 9,13-Aza-15-hydroxy-18-oxa-10,14-oxo-3,19,19,19-tetra-phenyl-6-thia-3,16,16-trimethyl-nonadecansaeure
Ausbeute: 50 %; Schmp. 56-7 C;
$R_F^B = 0.89$.

15 q) 9 13-Aza-16,16-dimethyl-15-hydroxy-18-oxa-10,14-oxo-3,19,19,19-tetraphenyl-6-thia-nonadecansaeure
Ausbeute: 89 %; Schmp. 62 C (Ether/Ligroin);
$R_F^E = 0.90$.

**Beispiel 16**

**9,12-Aza-10-oxo-13-phenyl-6-thia-tridecansaeure**

Man refluxiert eine Loesung von 3.89 g (10.0 mmol) 9,12-Aza-10-oxo-13-phenyl-6-thia-tridecansaeureethylester in 25 ml 2 N HCl und 5 ml Dioxan 2 h, engt auf ein Drittel ein, versetzt mit Aceton und kuehlt ab. Die sich abscheidenden Kristalle werden aus Ethanol umkristallisiert: 2.2 g (61 %) Hydrochlorid vom Schmp. 105-6 C;
$R_F^B = 0.54$; $R_F^E = 0.51$.

**Beispiel 17**

**9,13-Aza-15,17-dihydroxy-10,14-oxo-6-thia-3,16,16-trimethyl-heptadecansaeure**

Zu einer Loesung von 0.84 g (2.00 mmol) 9,13-Aza-15,17-di-hydroxy-10,14-oxo-6-thia-3,16,16-trimethyl-heptadecansaeureethylester in 10 ml 0.1 M Phosphatpuffer vom pH = 8.0 gibt man bei Raumtemperatur 0.2 ml (= 200 Einheiten) Schweineleberesterase. Anschließend haelt man den pH-Wert durch Zugabe von 1 N NaOH zwischen 7.5 und 8.0, bis dieser sich nicht mehr veraendert.

Man extrahiert mit Ether, saeuert an bis pH = 4 und extrahiert kontinuierlich mit $CH_2Cl_2$. Das nach Einengen der $CH_2Cl_2$-Phase erhaltene Produkt wird mehrfach mit Ligroin durchgearbeitet:
0.50 g (63 %); zaehes Wachs; $R_F^C = 0.35$; $R_F^E = 0.57$.

In analoger Weise erhaelt man die folgenden 5-Thiopentan-saeuren:

17 a) 9,13-Aza-15,17-dihydroxy-16,16-dimethyl-10,14-oxo-3-phenyl-6-thia-heptadecansaeure
Ausbeute: 50 %; zaehes Oel;
$R_F^B = 0.76$; $R_F^C = 0.38$; $R_F^E = 0.61$.

17 b) 9,13-Aza-15,17-dihydroxy-16,16-dimethyl-10,14-oxo-6-thia- heptadecansaeure
Ausbeute: 25 %; zaehes Oel;
$R_F^C = 0.21$, $R_F^E$ 0.49.

**Beispiel 18**

**9,13-Aza-3,3-dimethyl-6,10,14-oxo-14-phenyl-6-thia-tetradecansaeure**

Zu einer geruehrten Suspension von 226 mg (1.04 mmol) NaJO. in 3 ml Wasser gibt man bei 0 C 380 mg (1.00 mmol) 9,13-Aza-3,3-dimethyl-10,14-oxo-14-phenyl-6-thia-heptadecansaeure und 0.2 ml Ethanol und ruehrt 20 h im Eisbad.

Danach fuegt man 5 ml Wasser zu, saugt ab und extrahiert das Filtrat mit $CH_2Cl_2$. Der nach dem Einengen

alsbald erstarrende Rueckstand wird aus Essigester umkristallisiert: 0.26 g (66 %);
Schmp. 73-7 C;
$R_F^A = 0.17$; $R_F^B = 0.59$.
In analoger Weise erhaelt man aus dem entsprechenden Thioether:
18 a) 9,13-Aza-6,10-14-oxo-3,3,16,16-tetramethyl-6-thia-heptadecansaeure
Ausbeute: 52 %; Schmp. 143-4 C (Ether);
$R_F^A = 0.20$; $R_F^B = 0.59$.
18 b) 9,13-Aza-6,10,14-oxo-3-phenyl-6-thia-heptadecansaeure
Ausbeute:. 80 %; Oel; $n^{30}_D = 1.5390$;
$R_F^A = 0.14$; $R_F^B = 0.54$.

**Beispiel 19**

**9,13-Aza-16,16-dimethyl-6,6,10,14-oxo-6-thia-heptadecan-saeure**

Man ruehrt eine Loesung von 346 mg (1.00 mmol) 9,13-Aza-16-16-dimethyl-10,14-oxo-6-thia-heptadecansaeure in 3.5 ml Essigsaeure und 0.32 g 30proz. $H_2O_2$ 1.5 h bei 90-100 C, engt im Vakuum ein und arbeitet mehrmals mit Ligroin und Ether durch: 300 mg (79 %); Schmp. 95-6 C;,
$R_F^A = 0.22$; $R_F^B = 0.58$.
In Analogie erhaelt man aus den entsprechenden Thioethern:
19 a) 9,13-Aza-3,14-diphenyl-3-methyl-6,6,10,14-oxo-6-thia-tetradecansaeure
Ausbeute: 84 %; zaehes Oel;
$R_F^A = 0.23$; $R_F^B = 0.72$.
15 b) 9,13-Aza-6,6,10,14-oxo-3-phenyl-6-thia-heptadecansaeure
Ausbeute: 74 %; Schmp. 116-7 C;
$R_F^A = 0.17$; $R_F^B = 0.61$.

**Patentansprüche**

1. Carbonsäure-Derivate der allgemeinen Formel I

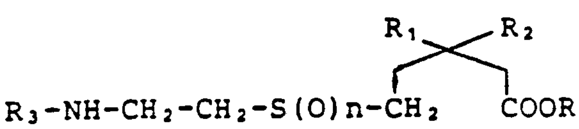

I

in der
R H, Alkyl, ein Metall-Kation, Ammonium, Alkylammonium
$R_1$ H, OH, Alkyl, O-Alkyl, O-Benzyl, O-Acyl
$R_2$ H, Alkyl, Aryl, Aralkyl
n 0, 1, 2
$R_3$ Acyl, wobei dieser Rest
a) eine geradkettige oder verzweigte, gesättigte oder ungesättigte Alkanoylgruppe mit 2 - 11 C-Atomen oder
b) einen Rest

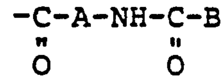

in dem A eine geradkettige oder verzweigte, gesättigte oder ungesättigte Alkylenkette mit 1 - 4 C-Atomen, die gegebenenfalls durch Hydroxy substituiert ist, und

B eine geradkettige oder verzweigte, gesättigte oder ungesättigte Alkylgruppe mit 1 - 8 C-Atomen, die gegebenenfalls ein- oder mehrfach durch Hydroxy, Carboxyl oder Phenyl substituiert ist, Phenyl oder Cycloalkyl oder

c) einen Rest

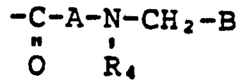

in dem A und B dieselben Gruppen wie unter b) und

$R_4$ H, Alkyl oder Aralkyl darstellen,

bedeutet, sowie deren pharmakologisch verträgliche Salze.

2. Carbonsäure-Derivate gemäß Anspruch 1, in denen R, $R_3$ und n die angegebene Bedeutung haben, $R_1$ Hydroxyl oder Methyl und $R_2$ Methyl bedeuten.

3. Carbonsäure-Derivate gemäß Anspruch 1 oder 2, in denen

n = 0,

R Wasserstoff oder Methyl oder Ethyl

$R_1$ Hydroxyl oder Methyl,

$R_2$ Methyl

und

$R_3$ die Gruppe 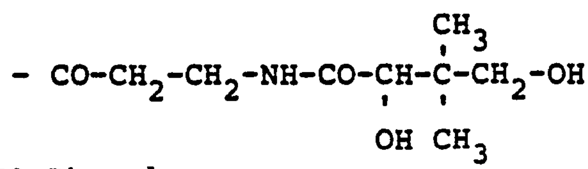

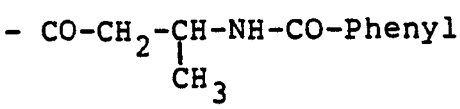

oder

$CO-CH_2-NH-CH_2$-Phenyl

darstellen.

4. Verfahren zur Herstellung von Carbonsäure-Derivaten der allgemeinen Formel I

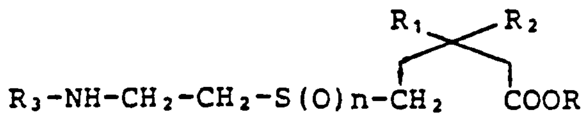

I

in der
R H, Alkyl, ein Metall-Kation, Ammonium, Alkylammonium
$R_1$ H, OH, Alkyl, O-Alkyl, O-Benzyl, O-Acyl
$R_2$ H, Alkyl, Aryl, Aralkyl
n 0, 1, 2
$R_3$ Acyl, wobei dieser Rest
a) eine geradkettige oder verzweigte, gesättigte oder ungesättigte Alkanoylgruppe mit 2 - 11 C-Atomen oder
b) einen Rest

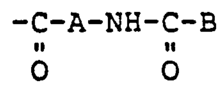

in dem A eine geradkettige oder verzweigte, gesättigte oder ungesättigte Alkylenkette mit 1 - 4 C-Atomen, die gegebenenfalls durch Hydroxy substituiert ist, und
B eine geradkettige oder verzweigte, gesättigte oder ungesättigte Alkylgruppe mit 1 - 8 C-Atomen, die gegebenenfalls ein- oder mehrfach durch Hydroxy, Carboxyl oder Phenyl substituiert ist, Phenyl -oder Cycloalkyl, oder
c) einen Rest

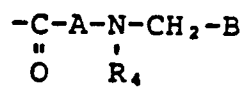

in dem A und B dieselben Gruppen wie unter b) und
$R_4$ H, Alkyl oder Aralkyl darstellen,
bedeutet, sowie deren pharmakologisch verträgliche Salze,
dadurch gekennzeichnet, daß man in an sich bekannter Weise
aa) eine Verbindung der Formel II
$R_3$-NH-$CH_2$-$CH_2$-X II
mit einer Verbindung der Formel III

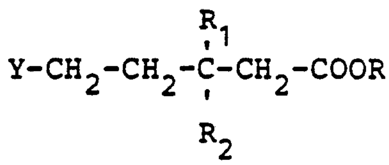

III

umsetzt,

wobei R, $R_1$, $R_2$ und $R_3$ die angegebene Bedeutung haben und X und Y einen reaktiven Rest darstellen, mit der Maßgabe, daß einer der beiden Reste eine -SH-Gruppe sein muß, oder

bb) eine Verbindung der Formel II

$R_3$-NH-$CH_2$-$CH_2$-X II

mit einer Verbindung der Formel IV

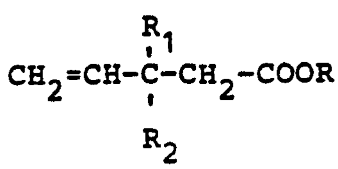

IV

umsetzt,

wobei R, $R_1$, $R_2$ und $R_3$ die angegebene Bedeutung haben und X eine SH-Gruppe darstellt, oder

cc) eine Verbindung der Formel V

$R_3$-Z V

mit einer Verbindung der Formel VI

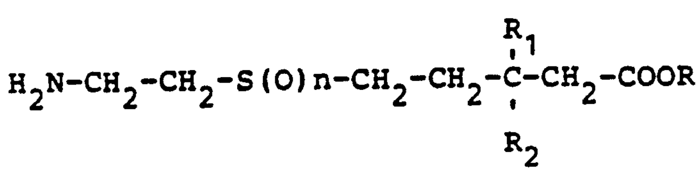

VI

umsetzt,

wobei R, $R_1$, $R_2$, $R_3$ und n die angegebene Bedeutung haben und Z eine reaktive Gruppe darstellt,

anschließend gewünschtenfalls Verbindungen der Formel I, in denen n = 0 oder 1 bedeutet, zu Sulfoxiden oder Sulfonen oxidiert, sowie die erhaltenen Verbindungen gewünschtenfalls in ihre pharmakologisch unbedenkliche Salze überführt.

5. Arzneimittel, enthaltend eine Verbindung gemäß Anspruch 1, 2 oder 3 und übliche Träger- und Hilfsstoffe.

6. Verbindungen gemäß Anspruch 1, 2 oder 3 zur Verwendung bei der Bekämpfung erhöhter Lipide im Blut.

**Claims**

1. Carboxylic acid derivatives of the general formula (I)

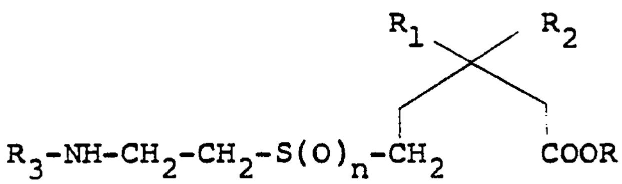

I

in which R signifies H, alkyl, a metal cation, ammonium, alkylammonium ion, $R_1$ H, OH, alkyl, O-alkyl, O-benzyl, O-acyl radical, $R_2$ H, alkyl, aryl, aralkyl radical, n 0, 1, 2, $R_3$ acyl, whereby this radical
a) is a straight-chained or branched, saturated or unsaturated alkanoyl group with 2 - 11 C-atoms or
b) is a radical

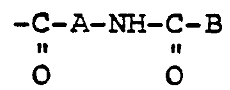

in which A is a straight-chained or branched, saturated or unsaturated alkylene chain with 1 - 4 C-atoms, which is optionally substituted by hydroxyl, and B is a straight-chained or branched, saturated or unsaturated alkyl group with 1 - 8 C-atoms, which is optionally substituted one or more times by hydroxyl, carboxyl or phenyl, phenyl or cycloalkyl or
c) is a radical

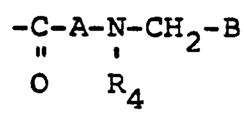

in which A and B represent the same groups as under b) and $R_4$ represents H, alkyl or aralkyl, as well as their pharmacologically acceptable salts.

2. Carboxylic acid derivatives according to claim 1, in which R, $R_3$ and n have the given meanings, $R_1$ signifies hydroxyl or methyl and $R_2$ methyl.

3. Carboxylic acid derivatives according to claim 1 or 2, in which n = 0, R represents hydrogen or methyl or ethyl, $R_1$ hydroxyl or methyl, $R_2$ methyl and $R_3$ the group

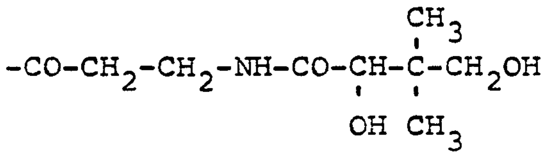 , 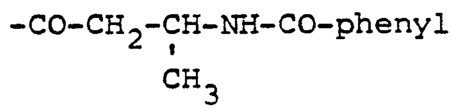

or $-CO-CH_2-NH-CH_2$-phenyl.

4. Process for the preparation of carboxylic acid derivatives of the general formula I

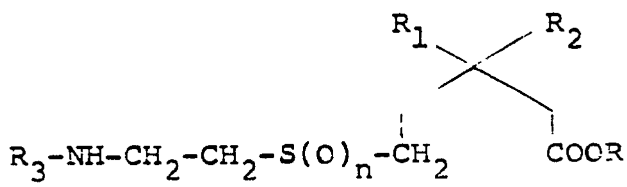

I

in which R signifies H, alkyl, a metal cation, ammonium, alkylammonium ion $R_1$ H, OH, alkyl, O-alkyl, O-benzyl, O-acyl radical, $R_2$ H, alkyl, aryl, aralkyl radical, n 0, 1, 2, $R_3$ acyl, whereby this radical

a) is a straight-chained or branched, saturated or unsaturated alkanoyl radical with 2 - 11 C-atoms or

b) is a radical

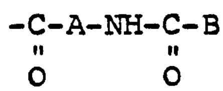

in which A is a straight-chained or branched, saturated or unsaturated alkylene chain with 1 - 4 C-atoms, which is optionally substituted by hydroxyl, and B is a straight-chained or branched, saturated or unsaturated alkyl group with 1 - 8 C-atoms, which is optionally substituted one or more times by hydroxyl, carboxyl or phenyl, phenyl or cycloalkyl, or

c) is a radical

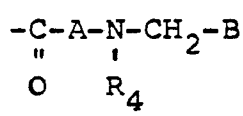

in which A and B represent the same groups as under b) and $R_4$ represents H, alkyl or aralkyl, as well as of their pharmacologically acceptable salts thereof, characterised in that, in per se known manner, one

aa) reacts a compound of the formula II
$R_3$-NH-$CH_2$-$CH_2$-X II
with a compound of the formula III

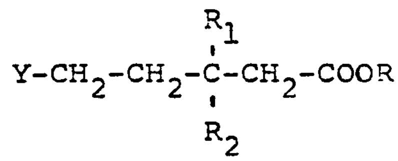

III

whereby R, $R_1$, $R_2$ and $R_3$ have the given meaning and X and Y represent a reactive residue, with the proviso that one of the two residues must be an -SH group, or
bb) reacts a compound of the general formula II
$R_3$-NH-$CH_2$-$CH_2$-X II
with a compound of the formula IV

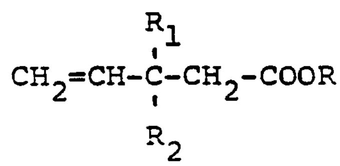

IV

whereby R, $R_1$, $R_2$ and $R_3$ have the given meaning and X represents an -SH group, or
cc) reacts a compound of the formula V
$R_3$ - Z V
with a compound of the formula VI

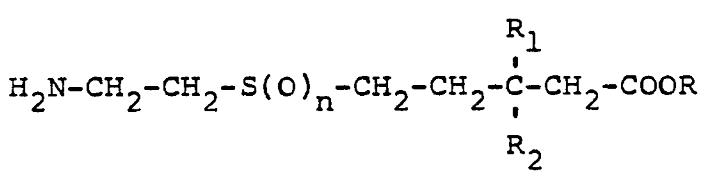

VI

whereby R, $R_1$, $R_2$, $R_3$ and $\underline{n}$ have the given meaning and Z represents a reactive group; whereafter, if desired, one oxidises compounds of the formula I in which $\underline{n}$ = 0 or 1, to sulphoxides or sulphones as well as, if desired, converts the compound obtained into their pharmacologically acceptable salts.

5. Medicaments, containing a compound according to claim 1, 2 or 3 and conventional carrier and adjuvant materials.

6. Compounds according to claim 1, 2 or 3 for use in the combating of increased lipids in the blood.

**Revendications**

1 - Dérivés de l'acide carbonique de formule générale I,

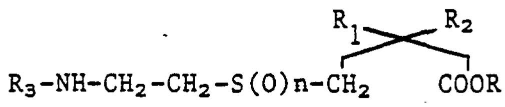 I

dans laquelle :
R représente H, un alkyle, un cation métal, de l'ammonium ou un alkylammonium
$R_1$, H, OH, un alkyle, O-alkyl, O-benzyl, O-acyl
$R_2$, H, un alkyle, un aryle, un aralkyle
n est égal à 0, 1, 2
$R_3$ représente un acyle, le reste étant:
a) un groupe alcanoyle saturé ou nonsaturé , à chaîne droite ou ramifiée, contenant de 2 à 11 atomes de C ou
b) un reste

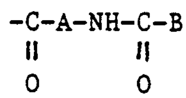

dans lequel A est une chaîne d'alkylène droite ou ramifiée saturée ou non saturée, contenant de 1 à 4 atomes de C, avec, éventuellement, substitution hydroxy,
et B, un groupe alkyle, à chaîne droite ou ramifiée, saturé ou non saturé , contenant de 1 à 8 atomes de C avec, éventuellement substitution simple ou multiple par hydroxy, carboxyle ou phényle, phényle ou cycloalkyle ou
c) un reste

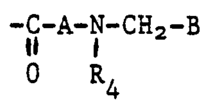

dans lequel A et B représentent les mêmes groupes que dans la rubrique b) et $R_4$ représente H, de l'alkyle ou de l'aralkyle, ainsi que leurs sels utilisables en pharmacologie.

2 - Dérivés de l'acide carbonique selon la revendication 1 dans lesquels R, $R_3$ et n ont les significations indiquées, $R_1$ representant de l'hydroxyle ou du méthyle et $R_2$ du méthyle.

3 - Dérivés de l'acide carbonique selon l'une des revendications 1 ou 2, dans lesquels :
n = 0
R représente de l'hydrogène ou du méthyle ou de l'éthyle
$R_1$ , de l'hydrogène ou du méthyle
$R_2$ , du méthyle et
$R_3$ représente le groupe

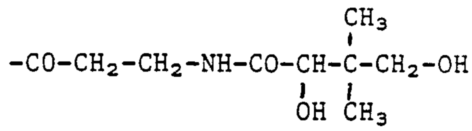 ;

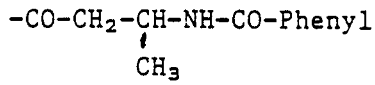 ou

$-CO-CH_2-NH-CH_3$ Phenyl

4 - Procédé de fabrication de dérivés de l'acide carbonique ayant pour formule générale :

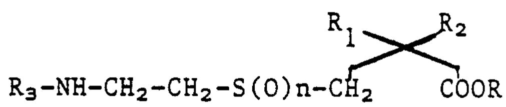 I

dans laquelle :
R représente H un alkyle un cation métallique, de l'ammonium, de l'alkylammonium
$R_1$, H, OH, un alkyle , O-alkyle , O-benzyle , O-acyle
$R_2$, H, un alkyle, un aryle, un aralkyle
n est égal à 0, 1, 2
$R_3$ représente un acyle, le reste étant
a) un groupe alcanoyle à chaîne droite ou ramifiée, saturé ou non saturé, contenant de 2 à 11 atomes de C ou
b) un reste

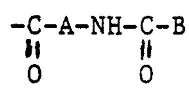

dans lequel A représente une chaîne d'alkylène droite ou ramifiée, saturée ou non saturée, contenant de 1 à 4 atomes de C, avec substitution éventuelle par de l'hydroxy.

B, un groupe alkyle à chaîne droite ou ramifiée, saturé ou non saturé , contenant de 1 à 8 atomes de carbone, avec substitution éventuelle, simple ou multiple, par hydroxy, carboxyle ou phényle, phényle ou cycloalkyle ou

c) un reste

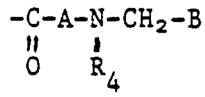

dans lequel A et B désignent les mêmes groupes que dans b), $R_4$ représentant H, un alkyle ou un aralkyle ainsi que leurs sels utilisables en pharmacologie, caractérisé en ce que, d'une manière connue, on fait réagir

aa) un composé de formule II

$R_3$-NH-$CH_2$-$CH_2$-X II

avec un composé de formule III

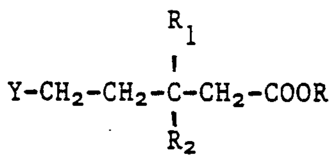

III

R, $R_1$, $R_2$ et $R_3$ ayant les significations déjà indiquées et X et Y représentant un reste réactif, avec la condition que l'un des deux restes doit être un groupe -SH- ou

bb) un composé de formule II

$R_3$-NH-$CH_2$-$CH_2$-X II

avec un compose de formule IV

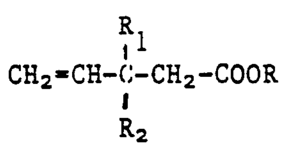

IV

R, $R_1$, $R_2$ et $R_3$ ayant les significations déjà indiquées et X représentant un groupe SH

cc) un composé de formule V

$R_3$-Z V

avec un composé de formule VI

$$H_2N-CH_2-CH_2-S(O)n-CH_2-CH_2-\underset{R_2}{\overset{R_1}{C}}-CH_2-COOR \qquad VI$$

R, $R_1$, $R_2$, $R_3$ et n ayant les significations déjà indiquées et Z représentant un groupe réactif,

des composés de formule I, pour lesquels n = 0 ou 1, pouvant être, à volonté, oxydés ensuite à l'état de sulfoxides ou de sulfones, les composés obtenus pouvant être transformés à volonté en leurs sels ne présentant pas d'inconvénients en pharmacologie.

5 - Médicament contenant un composé selon l'une des revendications 1, 2 ou 3 et les substances usuelles utilisées comme supports et produits d'appoint.

6 - Composés selon l'une des revendications I, 2 ou 3 destinés à être utilisés pour faire baisser le taux des lipides dans le sang.